# EUROPEAN PATENT SPECIFICATION

(11) **EP 1 344 491 B1**
(45) Date of publication and mention of the grant of the patent: **07.05.2008**
(21) Application number: 01997255.3
(22) Date of filing: 26.11.2001
(51) Int. Cl.: A61B 5/055, A61B 6/00

(54) **METHOD FOR TAKING CEREBRAL LAMINOGRAMS FOR WORKING UP LIMBIC SYSTEM**
VERFAHREN FÜR DIE AUFNAHME VON GEHIRNTOMOGRAMMEN ZUR UNTERSUCHUNG DES LIMBISCHEN SYSTEMS
PROCEDE POUR LAMINOGRAMMES CEREBRAUX POUR LE TRAITEMENT DU SYSTEME LIMBIQUE

(30) Priority: 27.11.2000 JP 2000359913
(43) Date of publication of application: 17.09.2003
(73) Proprietor: Matsuzawa, Taiju, Miyagi 989-3201 (JP)
(72) Inventor: Matsuzawa, Taiju, Miyagi 989-3201 (JP)
(74) Representative: Bannerman, David Gardner
(86) International application number: PCT/JP2001/010281
(87) International publication number: WO 2002/041775

(56) References cited:
- EP-A- 0 424 912
- US-A- 4 055 767
- US-A- 4 961 425
- US-A- 4 977 505
- US-A- 5 398 684
- TAIKI MATSUZAWA: 'Kino gazo keisoku ni yoru nou no hataraki no kaimei: MRI to PET ni yoru Alzheimer-byo no kaimei' SYSTEM/JOHO GODO SYMPOSIUM '97 1997, pages 15 - 17, XP002907854
- TORU HOSHIDA: 'MRI ni yoru kaima hentoutai no keisoku ni hitsuyouna kaibo' IGAKU NO AYUMI vol. 182, no. 3, 19 July 1997, pages 175 - 178, XP002907855
- SHOJI HIRANO ET AL.: 'Toubu MR zansouzo wo mochiita nou naibui no ichi sokutei' 1996NEN DENSHI JOHO TSUSHIN GAKKAI SOGO TAIKAI KOUEN RONBUNSHUU 11 March 1996, page D-108, XP002907856

## Description

The present invention relates to a novel method for forming cerebral laminograms.

In recent years, apparatuses for taking images for medical use have been widely developed ranging from those apparatuses that give images that can be directly seen after image taking to those apparatuses of the type in which computer-processed images are observed according to the development of digital image processing technology.

Magnetic resonance imaging apparatus (MRI), an X-ray computer tomographic apparatus (CT) or a positron emission tomographic apparatus (PET) is one of them.

Also, recently, an increase in demand for braindocks has led to frequent taking of tomography of particularly brains by use of the above-mentioned apparatus.

On the other hand, in recent years, the relationship between mental diseases and collapsed nerve nuclei in the limbic system of a brain has been increasingly unveiled ("Revolution in mind and brain", published by Tokuma Shoten Publishing Co., Ltd.). However, no method has been disclosed that prepares images of collapsed nerve nucleus in the limbic system with good reproducibility.

Therefore, when a cerebral laminogram on the above-mentioned apparatus is taken by using the conventional picture-taking method, it is difficult to provide image information sufficient for the diagnosis and therapy of patients who suffer from diseases that will lead to mental disorders.

Accordingly, it has been demanded to provide a method for giving cerebral laminogram for working up the limbic system that can provide efficacious information on medical observations to patients who are suffering all the diseases that could lead to mental disorders, i.e., dementia, psychosomatic disorder, depression, and schizophrenia.

Therefore, an object of the present invention is to provide a method for forming cerebral laminograms in order to obtain cerebral laminograms for working up the limbic system that are effective for studying mental disorders.

That is, the inventor of the present invention has made extensive studies on cerebral laminograms of patients who suffer from various diseases that led to mental disorders. As a result, he has confirmed that for grasping the state of mental disorders, it is very important to obtain laminographic images of the hippocampus and the amygdala in the limbic system, the brain stem, and the cerebral cortex all in a single image plane.

Then, he has found that observation of the collapsed nerve nucleus in the limbic system on the above-mentioned laminograms containing all taken in a single image plane is particularly effective for leading the results to subsequent medical diagnosis and made extensive studies on means to obtain such laminograms, thereby accomplishing the present invention.

The present invention is as defined in the claims.
Fig. 1 is a sketch clarifying a relationship between respective sites of a brain and a picture-taking plane according to the present invention, for explaining a method for forming laminograms according to the present invention.
Fig. 2 is a drawing (photograph) showing an image for clarifying the relationship between the respective sites of the brain and the picture-taking plane according to the present invention, for explaining the method for forming laminograms according to the present invention.
Figs. 3(a) and 3(b) are drawings (photographs) of the laminogram of the limbic system obtained by the method of the present invention in comparison with the conventional method, Fig. 3(a) being a laminogram taking the hippocampus and the amygdala in the limbic system, the brain stem, and the cerebral cortex all in a single image plane, and Fig. 3(b) being a sketch for explaining the differences between the method of the present invention and the conventional method.
Fig. 4 is a sketch clarifying the relationship between the respective sites of the brain and a plane on which an image is taken by the conventional method in order to explain a conventional image-taking method.
Figs. 5(a) to 5(c) are cross-sectional views obtained when laminograms are taken by use of the conventional image-taking method (cf. C in Fig. 3(b)) from the back to the front of the brain.
Fig. 6 is a schematic diagram of the limbic system.
Fig. 7 is a schematic diagram showing an apparatus applicable to the present invention.
Fig. 8 is a structural diagram relating to an embodiment of an apparatus for forming an image according to the present invention.
Figs. 9(a) to 9(c) are photographs of cerebral laminograms obtained by being taken by MRI using the method of the present invention.
Figs. 10(a) to 10(c) are photographs of cerebral laminograms obtained by being taken by MRI using the method of the present invention.

Hereinafter, the present invention will be described in detail.

The method of forming an image according to the present invention is a method that involves selecting a plane in which laminographic images of the hippocampus and the amygdaloid body in the limbic system, the brain stem, and the cerebral cortex all can be taken in a single plane and performing picture-taking in that plane.

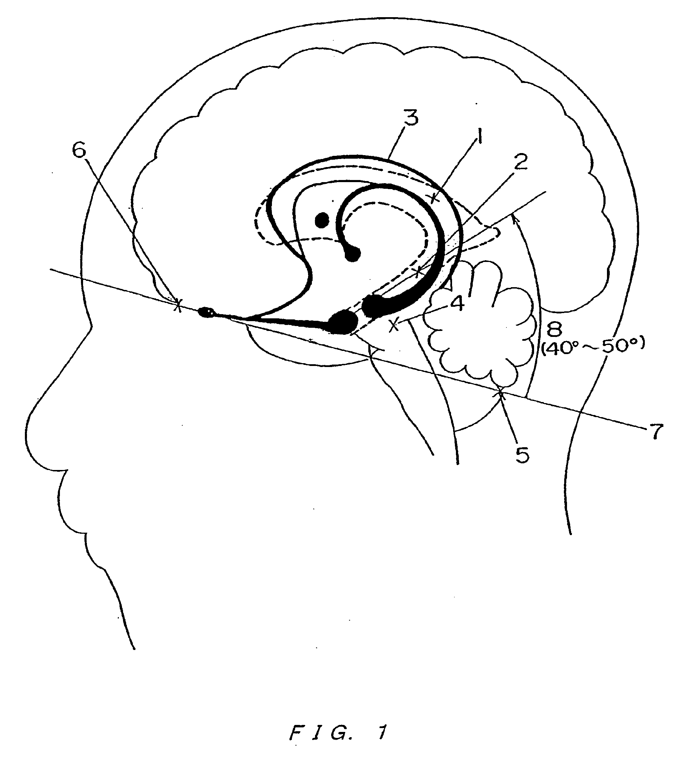

The method of forming an image according to the present invention will be described with reference to Fig. 1. In Fig. 1, reference numeral 1 denotes a lateral ventricle, reference numeral 2 denotes a lateral ventricle posterior horn (lateral posterior horn), reference numeral 3 denotes a limbic system, reference numeral 4 denotes a brain stem, reference numeral 5 denotes an inferior extremity of cerebellum, reference numeral 6 denotes an inferior extremity of a frontal lobe of cerebrum, reference numeral 7 denotes a plane linking the anterior inferior extremity of the frontal lobe and the inferior extremity of the cerebellum, and reference numeral 8 indicates an inclination of 40° to 50°.

In the present invention, cerebral laminograms are taken in a plane parallel to the reference numeral 2 (that is, a plane in which the image of the limbic system (having at least hippocampus and amygdala) and the images of the brain stem and the cerebral cortex all are taken in a single plane).

Further, according to the method of the present invention, the images of the limbic system (having at least the hippocampus and the amygdala), the brain stem and the cerebral cortex all can be obtained in a single plane as horizontally symmetrical images (as shown in the examples described hereinbelow).

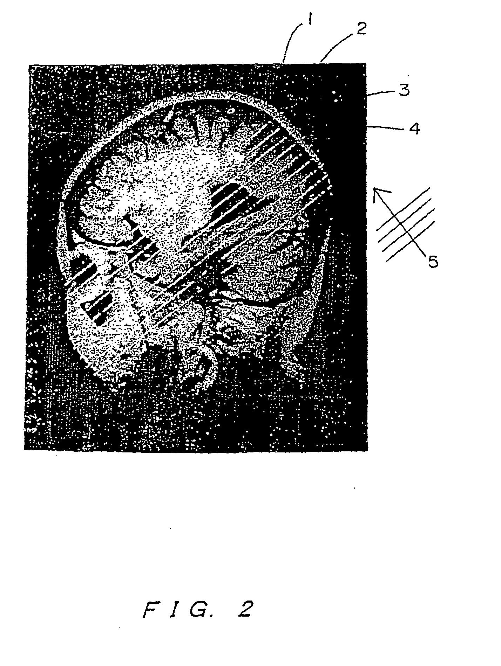

Furthermore, the method of the present invention is a method of obtaining laminograms by selecting a plane parallel to the plane where laminographic images of the hippocampus and the amygdala in the limbic system, the brain stem, and the cerebral cortex all can be obtained in a single plane (reference numeral 2 in Fig. 1) as a reference plane, and by moving the location of picture-taking such that pictures of plural planes parallel to the reference plane can be taken (cf. Fig. 2). In Fig. 2, reference numerals 1 to 4 denote planes parallel to the plane where the laminographic images of the hippocampus and the amygdala in the limbic system, the brain stem, and the cerebral cortex all can be taken in a single plane, respectively. Reference numeral 5 indicates the direction of movement of picture-taking locations.

Hereinafter, the present invention will be described in more detail.

A laminogram is taken in a plane parallel to the lateral ventricle posterior horn starting from the posterior extremity of lateral ventricle that contains cerebrospinal fluid (water of life) and extending linearly forward and downward along the hippocampus and the amygdala in the limbic system (cf. reference numeral 2 in Fig. 1).

However, in order to obtain a desired laminogram, a dozen or so laminograms parallel to the above-mentioned lateral ventricle posterior horn should preferably be taken (reference numerals 1 to 4 in Fig. 2).

This enables taking laminographic images of a three-layered structure consisting of the image of the limbic system (having at least the hippocampus and the amygdala) and the images of the brain stem and the cerebral cortex all in a single image as a horizontally symmetric image.

Then, based on the image information, a clear finding as to whether or not there is abnormality in the brain can be obtained.

Note that in the case where picture-taking is performed while moving the cross-section at which a picture is taken in the direction shown by the arrow designated by reference numeral 5 in Fig. 2, the proportion of the case where the above-mentioned three-layered structure is obtained as a single image is in fact about 3 to 4 sheets out of a dozen or so laminograms.

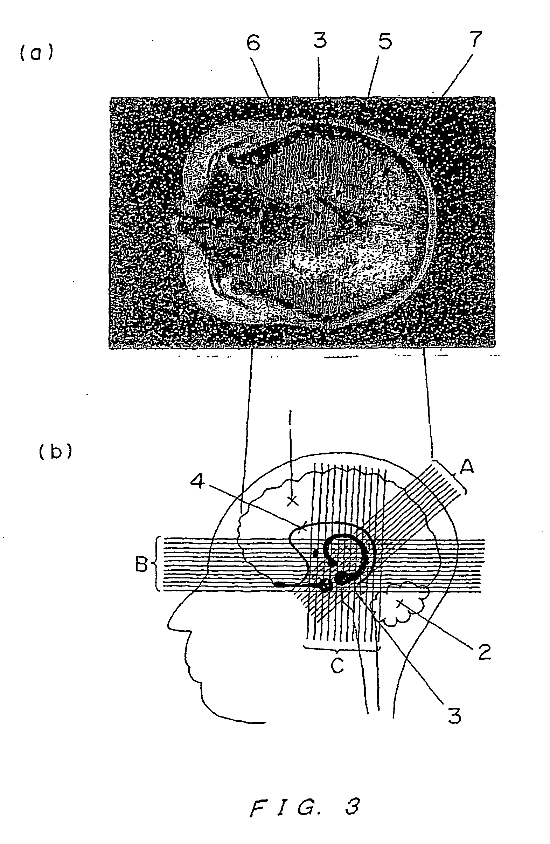

Fig. 3(a) is a photograph showing the laminographic images of the hippocampus and the amygdala in the limbic system, the brain stem, and the cerebral cortex all in a single image plane on an MRI apparatus by the method of the present invention. Further, Fig. 3(b) is a diagram that explains the differences between the method of the present invention and the conventional method.

In Fig. 3(a), reference numeral 3 denotes the brain stem, reference numeral 5 denotes the hippocampus, reference numeral 6 denotes the amygdala, and reference numeral 7 denotes the cerebral cortex.

In Fig. 3(b), reference numeral 1 denotes the cerebrum, reference numeral 2 denotes the cerebellum, reference numeral 3 denotes the brain stem, reference numeral 4 denotes the limbic system, symbol A indicates the direction of picture-taking by the method of the present invention, symbol B indicates the direction of picture-taking by the conventional method (1) and symbol C indicates the direction of picture-taking by the conventional method (2).

A preferred embodiment of the present invention will be described with reference to Figs. 3(a) and 3(b).

The reference numerals 1 to 4 in Fig. 2 correspond to the symbol A in Fig. 3(b).

As indicated by the symbol A in Fig. 3(b), taking a dozen or so laminograms parallel to the lateral ventricle posterior horn gives rise to the image shown in Fig. 3(a). As shown in this figure, laminographic images of a three-layer structure consisting of the image of the limbic system (having at least the hippocampus and the amygdala) and the images of the brain stem and the cerebral cortex are taken all in a single image in a horizontally symmetrical form.

By using this image, tests of the limbic system can be performed. In particular, since the collapsed nerve nuclei in the limbic system can be confirmed sufficiently, the findings can be applied to the diagnosis and therapy of diseases that cause mental disorders, for example, dementia, psychosomatic disorder, depression, and schizophrenia. In particular, these diseases manifest themselves as abnormalities in horizontally symmetrical patterns in the image of the limbic system as generally shown in the image in Fig. 3(a) and hence it is particularly effective in obtaining information of the diagnosis or therapy that the method of the present invention provides laminograms as horizontally symmetrical images.

In the present invention, the plane indicated by reference numeral 2 in Fig. 1, can be obtained by tilting the plane connecting the anterior inferior extremity of the frontal lobe and the inferior extremity of the cerebellum (indicated by reference numeral 7 in Fig. 1) by about 40° to 50°, preferably by 40° to 46° (most people can locate within the range of 43° ± 3°) toward the upper side of the cerebellum (as shown by the arc indicated by reference numeral 8 in Fig. 1) although the angle is different depending on the person. Note that the plane denoted by reference numeral 7 constitutes a reference plane quite different from the reference plane called OM line described hereinbelow but is a quite new reference plane set in order to select the planes denoted by the symbol A according to the present invention.

In the moving step in the present invention, it is recommended that the moving be performed at a gap between adjacent slices (hereinafter referred to as a "slice width") of 5 to 8 mm thick separated with gaps 1 to 2 mm wide, more preferably 5 mm thick separated with gaps 1 mm wide.

Next, conventional picture-taking methods will be described.

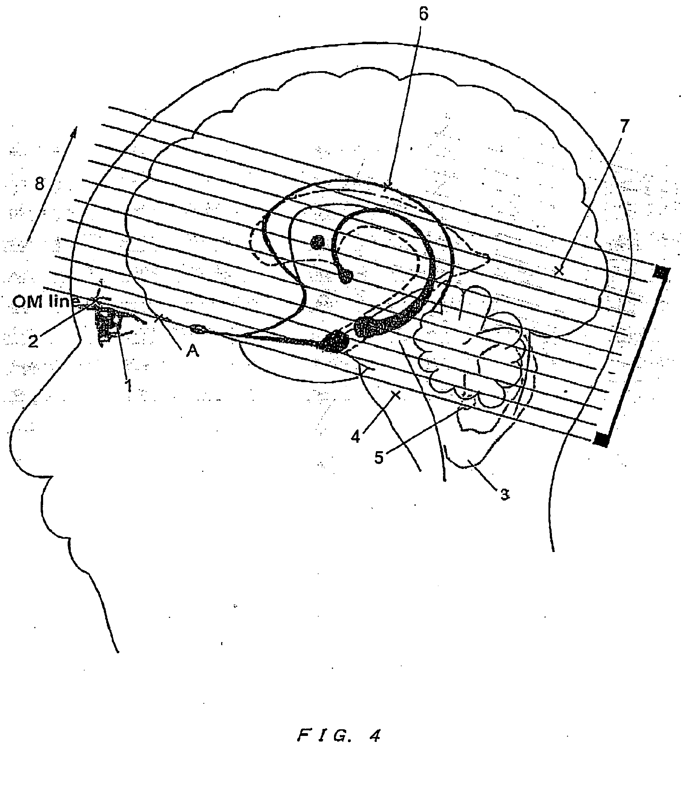

The conventional picture-taking method (1) is described with reference to Fig. 4. In Fig. 4, reference numeral 1 denotes an eye, reference numeral 2 denotes a superior margin of orbital, reference numeral 3 denotes an auricla, reference numeral 4 denotes a brain stem, reference numeral 5 denotes an external auditory meatus, reference numeral 6 denotes a limbic system, reference numeral 7 denotes plural planes parallel to the plane based on the line indicated by the symbol A, reference numeral 8 indicates a moving direction of the picture-taking, and symbol A indicates a line (OM line) connecting the superior margin of orbital to the external auditory meatus. The reference numeral 7 in Fig. 4 corresponds to the symbol B in Fig. 3(b).

As described above, the conventional picture-taking method (1) takes a plurality of images on planes parallel to the plane based on the OM line to obtain a laminogram of the brain.

However, this method cannot provide an image that contains laminographic images of the hippocampus and the amygdala in the limbic system, the brain stem, and the cerebral cortex all in a single image plane. In addition, the images of portions of the hippocampus and of the amygdala have to be taken in a plurality of cross-sections, so that it is difficult to grasp the sum total of the limbic system. Furthermore, originally, the images taken by the picture-taking method (1) are in many cases difficult to identify whether they are on the hippocampus or on the amygdala.

As described above, in the case where it is intended to perform examination on the limbic system based on image having taken a three-layer structure consisting of the image of the limbic system (having at least the hippocampus and the amygdala) and the laminographic images of the brain stem and the cerebral cortex all in a single image plane and obtain findings for medicine, in particular in the case where it is intended to obtain findings for medicine by taking the sum total of the limbic system and observing the collapsed nerve nuclei of the limbic system, the object cannot be achieved by the conventional method.

Also, the conventional picture-taking method (2) will be described.

This method involves taking images based on the conventional picture-taking method (1) described above in a plane perpendicular to the reference plane used therein. In Fig. 3(b), the symbol C indicates a direction of picture-taking in the conventional picture-taking method (2) that crosses perpendicularly a direction indicated by symbol B.

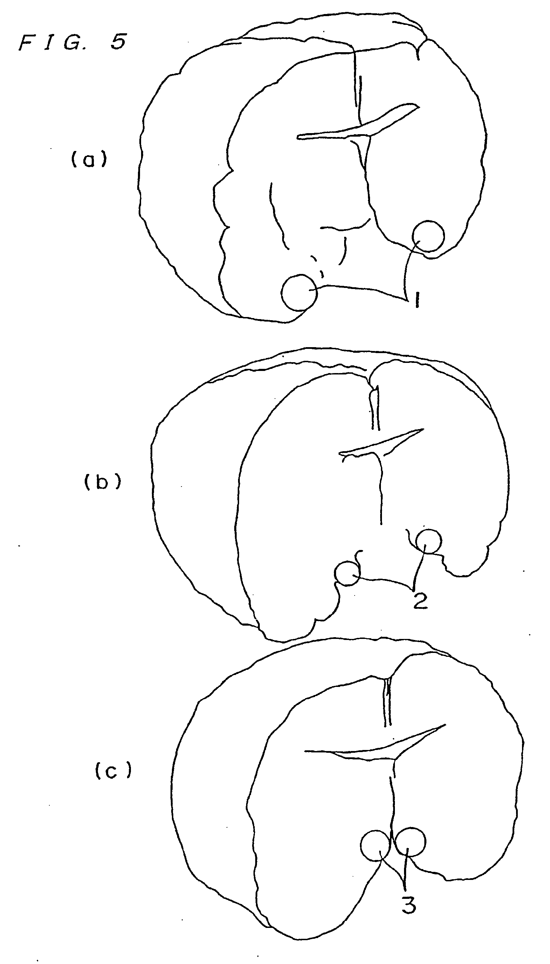

This method provides images that enable easy identification of the hippocampus or amygdala as compared with the conventional picture-taking method (1). However, as shown in Figs. 5, images of portions of the hippocampus and the amygdala are taken in a plurality of planes in cross-section. Figs. 5(a) to 5(c) are cross-sections of the brain obtained by taking laminograms from the back toward the front of the brain by the conventional picture-taking method (2). In Figs. 5, reference numeral 1 denotes the hippocampus, reference numeral 2 denotes the hippocampus and reference numeral 3 denotes the amygdala.

As described above, the picture-taking method (2) also fails to grasp the sum total of the limbic system and it can give only insufficient information in the case where it is intended to obtain findings for medicine by observation of abnormality in the limbic system and the state of destruction of the nerve nucleus.

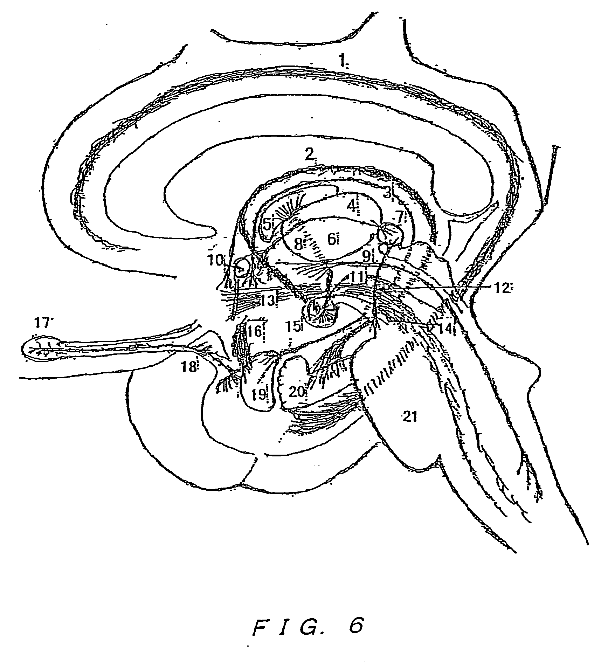

Note that the limbic system whose image is taken by the method of the present invention is a large terminal nerve nucleus like the hippocampus or amygdala in particular. However, it is more preferable that various other nerve nuclei (such as mammilary body, anterior nuclei of thalamus, medial nucleus of thalamus, and habenular nucleus) in the limbic system shown in Fig. 6 be taken. In Fig. 6, reference numeral 1 denotes a cingula (cingulative gyrus), reference numeral 2 denotes a fornix, reference numeral 3 denotes a terminal stria, reference numeral 4 denotes a medullary stria of thalamus, reference numeral 5 denotes an anterior nuclei of thalamus, reference numeral 6 denotes a medial nucleus of thalamus, reference numeral 7 denotes a cingula (cinguli), reference numeral 8 denotes a mammilary (body) thalamus tract, reference numeral 9 denotes (a Schuetz') dorsal longitudinal fasciculus, reference numeral 10 denotes an anterior commissure, reference numeral 11 denotes a mammilary (body) tegmentum tract, reference numeral 12 denotes habenular interpeduncular (nucleus) tract, reference numeral 13 denotes a medial telecephalon bundle (medial forebrain bundle), reference numeral 14 denotes a peduncle of mammilary body, reference numeral 15 denotes a mammilary body, reference numeral 16 denotes a leg loop, reference numeral 17 denotes an olfactory bulb, reference numeral 18 denotes an outer olfactory stria, reference numeral 19 denotes an amygdala (amygdaloid body) and reference numeral 20 denotes a hippocampus.

Further, in the method of the present invention, image-taking apparatuses for medicine of any type can be applied as far as they can take laminograms of a brain. Preferably, magnetic resonance imaging apparatus (MRI), X-ray computer tomographic apparatus (CT) or positron emission tomographic apparatus (PET) can be applied.

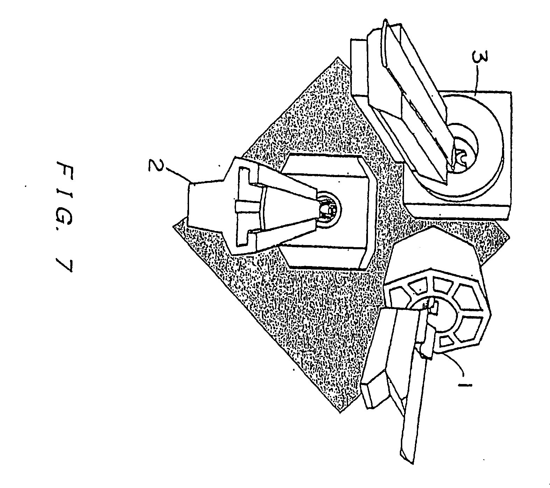

Next, an apparatus for forming images of the present invention will be described. As such apparatus is preferably used MRI, CT, or PET as described above. Fig. 7 is a schematic drawing showing these apparatuses. In Fig. 7, reference numeral 1 denotes an MRI apparatus, reference numeral 2 denotes a PET apparatus, and reference numeral 3 denotes an X-CT apparatus.

A commercially available apparatus as shown in Fig. 7 can be used in the present invention. Moreover, any apparatus that has means for forming laminograms of the hippocampus and the amygdala in the limbic system, the brain stem, and the cerebral cortex all in a single image plane, which is the feature of the method of forming images according to the present invention may be used without limitation.

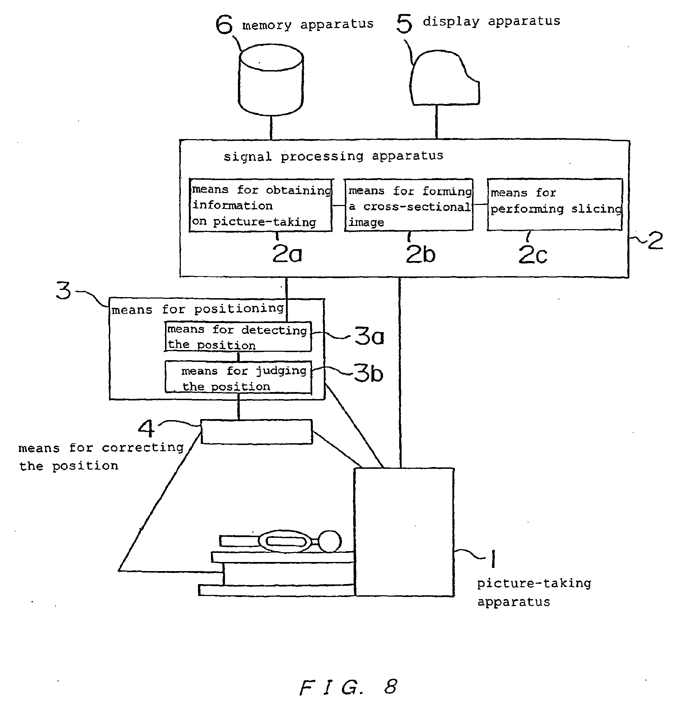

Fig. 8 is a structural diagram of an image-forming apparatus suitable for carrying out the method of the present invention. In Fig. 8, reference numeral 1 denotes a picture-taking apparatus, reference numeral 2 denotes a signal processing apparatus, reference numeral 2a denotes means for obtaining information on picture-taking, reference numeral 2b denotes means for forming a cross-sectional image, reference numeral 2c denotes means for performing slicing, reference numeral 3 denotes means for positioning, reference numeral 3a denotes means for detecting the position, reference numeral 3b denotes means for judging the position, reference numeral 4 denotes means for correcting the position, reference numeral 5 denotes a display apparatus, and reference numeral 6 denotes a memory apparatus.

As shown in Fig. 8, the image-forming apparatus includes a picture-taking apparatus unit that takes an image of a brain (1), a signal processing apparatus unit (2) that performs signal processing of the thus-taken information, a display apparatus unit (5) that displays the processed information, and a memory apparatus unit (6) that stores the processed information. The above-mentioned signal processing apparatus unit (2) further has means for obtaining picture-taking information (2a), means for taking a laminogram (2b), and means for performing slicing (2c) and so forth. These means are realized in a central processing unit of the computer.

Furthermore, the apparatus has means for positioning (3) and means for correcting the position (4) in order to take a desired image. The means for positioning (3) has a detection means (3a) for detecting the information of a brain laminogram for performing the positioning and a judgment means (3b) for judging the positional relationship between the laminogram of the brain and the direction of picture-taking (the inclination of the picture-taking direction to the brain) based on the obtained information. The means for correcting the position (4) has a function to correct the inclination of the direction of picture-taking in accordance with the content of the above-mentioned judgment.

The means for positioning (3) and the means for correcting the position (4) may be provided as separate apparatuses or may be incorporated in other apparatuses such as the signal processing apparatus unit (2).

### Examples

Hereinafter, the present invention will be described in more detail by examples.

### Example 1

1) A subject was laid on his or her back on a bed of an MRI apparatus, with the head being placed in a headrest (a fixing apparatus placing the head therein).
2) The chin and the forehead were fixed with a fixing band and the body was fixed with a body fixing belt.
3) A head coil was placed on the head.
4) The bed was moved and the root of nose portion was positioned at the end of the magnet by using a positioning light.
5) The bed was further moved in the magnet of the machine so that the center of the magnetic field corresponded to the root of nose portion (this action was incorporated in the apparatus). This allowed the root of nose portion to be placed in the center of picture-taking.
6) By utilizing a head-sagittal section-positioning machine (having the functions of the above-mentioned means for positioning (3) and the means for correcting the position (4) in combination), positioning was performed. On this occasion, the focus was once centered on the plane linking the anterior inferior extremity of the frontal lobe and the inferior extremity of the cerebellum, and then, the lateral ventricle posterior horn located within the range of 40° to 50° (in the case of most people, it being possible to set this within the range of 43° ± 3°) toward the upper portion of the cerebellum (in left-handed movement) was searched and a plane parallel to the lateral ventricle posterior horn was taken as a reference.
   Then, a dozen or so each images of a thickness of a slice of 6 mm and an interval of 2 mm and of a thickness of a slice of 5 mm and an interval of 1 mm from the reference plane were taken.
7) The above-mentioned picture-takings were performed in two image types, i.e., Tl-enhanced image and T2-enhanced image. These were taken in number of a dozen or so. Among them, those laminographic images in which the image of the limbic system (having at least the hippocampus and the amygdala), and the laminographic images of the brain stem and of the cerebral cortex all were taken in a single image plane were selected. Three or four sheets of satisfactory images were obtained for each of T1 and T2.

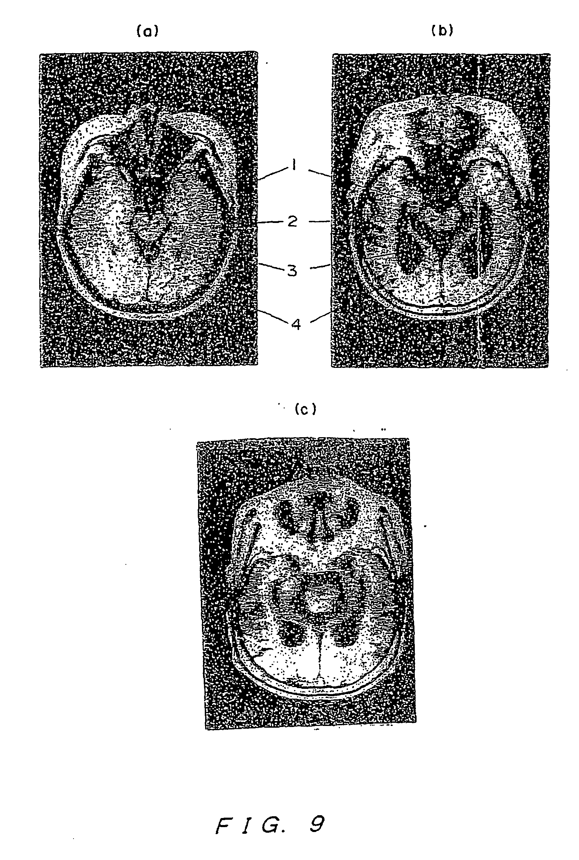

The results of picture-taking are shown in Figs. 9. Figs. 9 are photographs of the laminograms of a brain taken by MRI.

Fig. 9(a) is the laminogram of the brain of a healthy person, Fig. 9(b) is the laminogram of the brain of a person suffering from Alzheimer's disease (intermediate stage), and Fig. 9(c) is the laminogram of the brain of a person suffering from multi-infarct dementia. In Figs. 9, reference numeral 1 denotes an amygdala, reference numeral 2 denotes a brain stem, reference numeral 3 denotes a hippocampus, and reference numeral 4 denotes a cerebral cortex.

The results revealed that in Alzheimer's disease, the limbic system is selectively destructed (apoptosis; cell suicide). In Alzheimer's disease, collapse of the limbic system including major nerve nuclei such as hippocampus and amygdala proceeds from the back toward the front quite horizontally symmetrically as in the case of a destructive experiment. As the condition proceeds, first, the association areas (temple, vertex, and occiput) in the posterior of the cerebral cortex fall in functional disorder, and then, the association areas (temple and motor areas) in the anterior of the cerebral cortex are affected in order.

Alzheimer's diseases have been studied centered on the abnormality in the cerebral cortex only. However, this idea is not correct. As shown in Fig. 9(b), it becomes essentially important to know the state of collapse of nerve nuclei in the limbic system on considering the onset of the Alzheimer's disease.

Fig. 9(c) shows a case of early multi-infarct dementia. The atrophy of the hippocampus and amygdala is obvious like the Alzheimer's disease. For the deficit of the nerve cell in dementia shown in Figs. 9(b) and 9(c), the fundamental therapy of the disease is probably possible by performing a therapy such as injection of ES cells (embryonic stem cells).

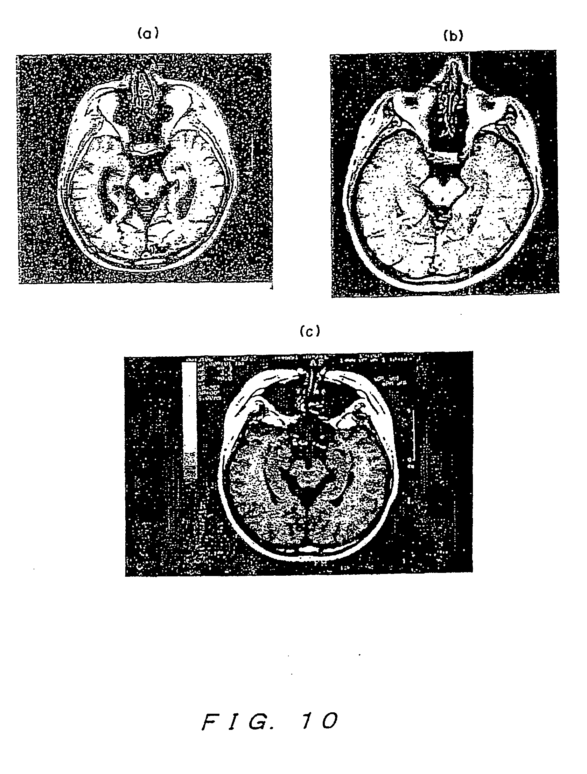

Furthermore, photographs of laminograms of brains of patients suffering various diseases causing various mental disorders obtained by the method of the present invention are shown. Fig. 10(a) is a laminogram of the brain of a child (13-year-old male) suffering from psychosomatic disorder, Fig. 10(b) is a laminogram of the brain of a women (40 years old) suffering from depression, and Fig. 10(c) is a laminogram of the brain of a male (19 years old) suffering from schizophrenia.

As shown in Figs. 9 and Figs. 10, the present invention has revealed that all the diseases accompanied by psychological disorders show the collapsed nerve nuclei in the limbic system.

In addition, it is extremely characteristic that the state of collapse occurs always in a horizontal symmetry.

The present invention can be said to be an invention that has an important significance, giving a clue to the scientific elucidation of mental diseases whose causes have hitherto been quite unknown.

Therefore, according to the present invention, it is possible to provide a method for forming cerebral laminograms in order to obtain cerebral laminograms for working up the limbic system that are effective for studying mental disorders.

## Claims

1. A method for forming a cerebral laminogram comprising steps of:
setting a reference plane parallel to a single plane in which images of hippocampus and amygdala in the limbic system, brain stem, and cerebral cortex are included, and
taking a laminogram on said single plane parallel to the reference plane by moving the position for taking the laminogram in a direction transverse to the reference plane,
wherein a plane parallel to the lateral ventricle posterior horn, starting from a posterior extremity of the lateral ventricle and extending linearly in a forwards and downwards direction along the hippocampus and amygdala in the limbic system, is set as the reference plane.

2. The method for forming a cerebral laminogram according to claim 1, wherein the laminogram is taken so that it is in a horizontally symmetrical form with respect to the centre thereof.

3. The method for forming a cerebral laminogram according to claim 1 or 2, wherein a plurality of laminograms in different planes is obtained by taking a plurality of images from different positions.

4. The method for forming a cerebral laminogram according to any one of claims 1 to 3, wherein a plane tilted by 40° to 50° from the plane connecting an anterior inferior extremity of the frontal lobe and an inferior extremity of the cerebellum toward an upper side of the cerebellum is set as the reference plane.

## Patentansprüche

1. Verfahren zum Bilden eines zerebralen Laminogramms mit den Schritten:
Setzen einer Referenzebene parallel zu einer einzelnen Ebene, in der Bilder eines Hippokampus und einer Amygdala in dem limbischen System, ein Hirnstamm und ein zerebraler Cortex liegen, und
Aufnehmen eines Laminogramms in der einzelnen Ebene parallel zu der Referenzebene durch ein Bewegen der Position zum Aufnehmen des Laminogramms in einer Richtung schräg zur Referenzebene,
wobei eine Ebene parallel zu dem Hinterhorn eines Seitenventrikels als die Referenzebene eingestellt wird, die an einer hinteren Extremität des Seitenventrikels beginnt und sich linear in einer Vorwärts- und Abwärtsrichtung entlang des Hippocampus und der Amygdala in dem limbischen System erstreckt.

2. Verfahren zum Bilden eines zerebralen Laminogramms nach Anspruch 1, wobei das Laminogramm so aufgenommen wird, dass es bezogen auf sein Zentrum eine horizontal symmetrische Form hat.

3. Verfahren zum Bilden eines zerebralen Laminogramms nach Anspruch 1 oder 2, wobei eine Vielzahl von Laminogrammen in verschiedenen Ebenen durch Aufnehmen einer Vielzahl von Bildern aus verschiedenen Positionen aufgenommen wird.

4. Verfahren zum Bilden eines zerebralen Laminogramms nach einem der Ansprüche 1 bis 3, wobei eine um 40° bis 50° aus der Ebene, die eine vordere untere Extremität des frontalen Lappens und eine untere Extremität des Kleinhirns in Richtung einer oberen Seite des Kleinhirns verbindet, geneigte Ebene als die Referenzebene eingestellt wird.

## Revendications

1. Procédé destiné à former un laminagramme cérébral comprenant les étapes consistant à :
régler un plan de référence parallèle à un plan unique dans lequel sont incluses des images de l'hippocampe et de l'amygdale du cervelet dans le système limbique, le tronc cérébral et le cortex cérébral, et
réaliser un laminagramme sur ledit plan unique parallèle au plan de référence en déplaçant la position pour réaliser le laminagramme dans une direction transversale au plan de référence,
dans lequel un plan parallèle à la corne postérieure de ventricule latéral, au départ d'une extrémité postérieure du ventricule latéral et s'étendant linéairement dans une direction vers l'avant et vers le bas le long de l'hippocampe et de l'amygdale du cervelet dans le système limbique, est réglé en tant que le plan de référence.

2. Procédé destiné à former un laminagramme cérébral selon la revendication 1, dans lequel le laminagramme est réalisé de sorte qu'il soit sous une forme horizontalement symétrique par rapport à son centre.

3. Procédé destiné à former un laminagramme cérébral selon la revendication 1 ou 2, dans lequel une pluralité de laminagrammes dans des plans différents sont obtenus en réalisant une pluralité d'images à partir de positions différentes.

4. Procédé destiné à former un laminagramme cérébral selon l'une quelconque des revendications 1 à 3, dans lequel un plan incliné de 40° à 50° à partir du plan reliant une extrémité inférieure antérieure du lobe frontal et une extrémité inférieure du cervelet, vers un côté supérieur du cervelet, est réglé en tant que le plan de référence.
